Europäisches Patentamt

European Patent Office

Office européen des brevets

(11) Publication number: **0 464 905 A1**

# EUROPEAN PATENT APPLICATION

(21) Application number: **91201576.5**

(22) Date of filing: **20.06.91**

(51) Int. Cl.⁵: **C12P 41/00, C12P 17/02**

(30) Priority: **25.06.90 NL 9001443**

(43) Date of publication of application:
**08.01.92 Bulletin 92/02**

(84) Designated Contracting States:
**BE CH DE DK ES FR GB IT LI NL SE**

(71) Applicant: **Naamloze Vennootschap DSM
Het Overloon 1
NL-6411 TE Heerlen(NL)**

(72) Inventor: **Geerlof, Arie
Rembrandtlaan 62
NL-2406 HB Alphen a/d Rijn(NL)**
Inventor: **Duine, Johannis Adriaan
Meeuwensingel 70
NL-3121 XM Schiedam(NL)**

(74) Representative: **Jacobs, Monique S.N. et al
Octrooibureau DSM Postbus 9
NL-6160 MA Geleen(NL)**

(54) **Enzymatic, enantioselective preparation of R-glycidol.**

(57) Enzymatic, enantioselective preparation of R-glycidol by subjecting a mixture of R,S-glycidol to the effect of a suitable enantioselective oxidizing enzyme.

Suitable enzymes are alcohol dehydrogenases, in particular PQQ dependent alcohol dehydrogenases. In this manner R-glycidol with a high e.e. can be prepared with relatively low conversion.

EP 0 464 905 A1

The invention relates to the enzymatic, enantioselective preparation of R-glycidol.

Such a process is known from US-A-4732853, which describes the enzymatic hydrolysis of (R,S)-glycidyl esters with the use of a lipase.

In that process, however, an ester of glycidol is used as starting material. This ester has to be initially prepared from glycidol, so that an extra step in the preparation is required. Moreover, the lipase yields R-glycidol of poor optical purity.

The object of the invention is to provide a simple process wherein the desired enantiomer is already present in the mixture of enantiomers which constitutes the starting material.

This is achieved according to the invention by treating a mixture of R- and S-glycidol with a suitable enantioselective oxidizing enzyme.

Optically pure R-glycidol as well as derivatives thereof, such as for instance the tosyl derivative, are suitable for a variety of applications, in particular for use as starting materials for the preparation of chiral pharmaceutical compounds derived therefrom. A general condition, however, is that the product should be sufficiently enantiomerically pure, which usually means that mostly an enantiomeric excess of more than 98% is required. For this reason there is much interest in alternative processes for the manufacture of these key chiral synthons.

The publication of T. Katsuki and K.B. Sharpless in J. Am. Chem. Soc. 102, 5974-5976 (1980) describes of a chemical process for the preparation of chiral glycidols by a chemical route using asymmetric epoxides of allyl alcohol. Such a chemical route is mostly less attractive in practice because of the scaling-up problems and the need to use extremely dry conditions to avoid poisoning of the catalyst.

The present invention is based on an enantioselective enzymatic oxidation of R,S-glycidol, with highly preferential oxidation of the S-enantiomer, thus leaving an R-glycidol enriched product.

Highly suitable organisms for the present invention are for instance Acetobacter pasteurianus, Acetobacter aceti and Gluconobacter oxydans. These organisms are produced in aerated media which contain a carbon source, a nitrogen source, vitamins, minerals, and/or the like. The culture can be grown as a batch, fed batch or continuous process.

Such culture methods are generally known and have been described frequently in patent specifications and scientific publications, such as for example EP-A-244912, so that a description thereof is superfluous here. The enzyme preparation as used in the present invention may be a crude enzyme solution or a purified enzyme, but it may also consist of (permeabilized and/or immobilized) cells possessing the desired activity or of a homogenate of cells with such activity. The enzyme can also be used in an immobilized form or in a chemically modified form. If there is also any undesired antagonist activity present in the enzyme preparation used, it is advisable to remove the undesired activity or to suppress it in order to obtain the maximum enantioselectivity. Therefore the invention is not restricted in any way by the form in which the enzyme is used or restricted by the enzyme purity. It is also possible of course within the framework of the invention to use an alcohol dehydrogenase, preferably a PQQ dependent (PQQ stands for pyrrolo-quinoline-quinone, that is 2,7,9-tricarboxy-1H-pyrrolo[2,3f]quinoline-4,5-dione) alcohol dehydrogenase, originating from a mutant or a genetically modified microorganism.

If insufficient or no PQQ is formed during the culture of the microorganism, it may be advantageous to add extra PQQ before the oxidation. Instead of PQQ, PQQ analogues may be used, such as for example monoesters of PQQ (on the 2 position), 3-methyl-PQQ, 3-ethyl-PQQ, 3-propyl-PQQ, N-methyl-PQQ, N-ethyl-PQQ, 8-methyl-PQQ, 4-hydroxy-PQ (PQ stands for pyrroloquinoline) or 5-hydroxy-PQ after oxidation, or the PQQ/acetone adduct and other aldehyde and ketone adducts, as described by J.A. Jongejan, B.W. Groen and J.A. Duine in "PQQ and Quinoproteins" (J.A. Jongejan and J.A. Duine, eds.), Kluwer Academic Publishers, Dordrecht, 1989, 205-216.

For enantioselective oxidation of R-glycidol, use is preferably made of a PQQ dependent alcohol dehydrogenase originating from Acetobacter pasteurianus, ATCC 12874, Acetobacter aceti, ATCC 15973, or Gluconobacter oxydans, ATCC 621.

Enantioselective enzymatic oxidations are known as such. Enantioselective oxidation of glycidol, however, has not been described earlier in the literature. The enantioselectivity is based on a difference in the rates of conversion of the various enantiomers. The reaction products obtained are enriched in one of the enantiomers. For practical purposes it is in general desirable to obtain one of the enantiomers in large excess. This is achieved by stopping the conversion at a certain stage of conversion. For enantioselective, enzymatic hydrolyses this has been described by Qu-Ming et al. in Tetrahedron Letters 27 (1986) 5203 ff. and more in general by Ghing-Shih Chen et al. in J. Am. Chem. Soc. 104 (1982) 7294 ff. The general theory of enantioselective conversions described in those publications also applies to the present process.

According to the present process the effect of an alcohol dehydrogenase, preferably a PQQ dependent alcohol dehydrogenase such as an alcohol dehydrogenase from the group of acetic acid bacteria, causes the S-enantiomer of glycidol to be oxidized faster than the R-enantiomer, as a result of which an R-glycidol enriched reaction product remains. There appears to be considerable difference between the reaction rates of the enantiomers, so that, starting from a racemic mixture, already at a conversion rate of 55% an enantiomeric excess of more than 95% R-glycidol can be obtained. An enantiomeric excess of more than 99% can already be obtained, also starting from a racemic mixture, at a conversion between 55 and 60%. Starting from R-enantiomer enriched mixtures, better results are obtained, with the enzyme preserving its selectivity. The R- and S-enantiomer percentages in the end product shift according to general theory, depending on the R- and S-enantiomer percentages in the starting product.

The cells used in the reaction can be recovered if desired and used again in a following conversion, without any significant loss of activity.

The reactions are usually carried out in an aqueous medium. It is also well possible to carry out the oxidation/reduction reactions in organic solvents.

The pH of the reaction mixture is mostly between 3 and 10, preferably between 4 and 7. The pH can be kept at a certain value by for instance adding a buffer or by continuously adding an alkaline solution.

The temperature at which the reaction is carried out can be varied within wide limits and is generally between -20°C and 100°C, preferably between 20 and 35°C.

The oxidation may be carried out by any available oxidation means. Preferably molecular oxygen is used. In order to ensure a good supply of oxygen for the oxidation, which may be in the form of air if desired, the reaction is mostly carried out with vigorous aeration. The reaction may also be carried out under electrochemical conditions.

The invention will be elucidated by the following examples without being restricted thereto.

The reaction product analyses in these examples, in particular with regard to determination of the enantiomeric excess (e.e.) were performed by the method described below (TAGIT method), the formulas of Chen et al. (J. Am. Chem. Soc. 104, 7294 ff (1982)) being optionally used for calculation of the selectivity value for the reaction. For determination of the conversion, use was made of column separation using an Aminex HPX-87H HPLC column, with 0.01N $H_2SO_4$ as mobile phase, and refractive index for detection of glycidol.

All analyses were performed on samples taken from the reaction mixture at different points in time, the quantities being 1-2 ml for determination of the conversion and 10-50 ml for the e.e. determination, depending on the conversion.

For the e.e. determination the samples, after being saturated with sodium chloride, were extracted twice with 20-100 ml dichloromethane. 50 $\mu$l of the residue obtained after evaporation of the solvent is dissolved in 1.0 ml n-butylamine. This solution is heated for 1 hour at 100°C in a closed reaction vessel. Next, the excess of n-butylamine is evaporated and the residue dried overnight in a vacuum desiccator over $P_2O_5$. This residue is dissolved in 0.5 ml acetonitrile, after which 2.5 $\mu$l of the resulting solution is mixed with 50 $\mu$l acetonitrile and 1 mg 2,3,4,6-tetra-O-acetyl-ß-glucopyranosylisothiocyanate (TAGIT). Five minutes after the mixing the e.e. determination is carried out with a reversed phase ($C_{18}$) column using 48/52 methanol/water as mobile phase.

This method appears to be very suitable for determination of the enantiomeric excess. It gives excellent separation of the R- and S-enantiomers of glycidol (in the form of derivatives).

Example I

Acetobacter pasteurianus ATCC 12874 was grown in a 2.0 litre Erlenmeyer (28°C, 250 rpm) containing 300 ml culture medium. The composition of this culture medium was as follows: 1.55 $gl^{-1}$ $K_2HPO_4$, 0.85 $gl^{-1}$ $NaH_2.PO_4.H_2O$, 2.0 $gl^{-1}$ $NH_4Cl$, 0.1 $gl^{-1}$ $(NH_4)_2SO_4$, 0.1 $gl^{-1}$ $MgCl_2.6H_2O$, 0.1 $gl^{-1}$ yeast extract, 0.2 ml trace elements solution (W. Vishniac & M. Santer Bact. Rev. (1957) 21, 195-213) and 10 $gl^{-1}$ ethanol; the pH was 7.0. At the end of the exponential growth phase, the cells were separated out by means of centrifugation.
The cells thus obtained were added to 100 ml 50 mM potassium phosphate buffer pH 6.0 (30°C) containing 100 mM R,S-glycidol. In order to ensure a sufficient supply of oxygen the incubation mixture was stirred and aerated. The pH was kept at 6.0 by titration with 2N NaOH. When 59% of the R,S-glycidol had been oxidized the incubation was stopped, the cells were separated out by centrifugation and the remaining alcohol in the water phase was analyzed for optical purity.
The enantiomeric excess amounted to 99%.

Example II

Acetobacter pasteurianus ATCC 12874 was grown batchwise as described in example I. The cells obtained were added to a 1.5- litre fermentor containing 1.0 litre 50 mM potassium phosphate buffer pH 6.0 and 100 mM R,S-glycidol. During the incubation the pH was kept at 6.0 by addition of 3.0 N NaOH, the temperature was 30°C and the dissolved oxygen concentration was kept at appr. 100 $\mu$M by adjusting both the stirring speed and the air supply. When 56% of the glycidol had been oxidized the incubation was stopped. The enantiomeric excess of R-glycidol was 98%.

Example III

As in example I, an R,S-glycidol oxidation experiment was performed with A. pasteurianus ATCC 12874 at 20˚C and pH 6.0. After oxidation of 64% of the alcohol the reaction was stopped. The remaining glycidol contained R-glycidol with an enantiomeric excess of 97.5%.

Example IV

As in example I, an R,S-glycidol oxidation experiment was performed with A. pasteurianus ATCC 12874 at 20˚C and pH 5.0. After oxidation of 63% of the alcohol the reaction was stopped. The remaining glycidol contained R-glycidol with an enantiomeric excess of 98.5%.

Example V

Acetobacter aceti ATCC 15973 was grown batchwise as described above for Acetobacter pasteurianus ATCC 12874. The cells obtained were added to 100 ml 50 mM potassium phosphate buffer pH 6.0 (20˚C) containing 100 mM R,S-glycidol. In order to ensure a sufficient supply of oxygen the incubation mixture was stirred and aerated. The pH was kept at 6.0 by titration with 2N NaOH. When 64% of the glycidol supplied had been oxidized the incubation was stopped and the cells were separated out by centrifugation. The resulting supernatant contained R-glycidol with an enantiomeric excess of 96%.

Example VI

Membrane-bound, PQQ dependent alcohol dehydrogenase of Gluconobacter oxydans ATCC 621 (obtained as described in Agric. Biol. Chem. 42, 2045-2056 (1978)) was added to 20 ml potassium phosphate buffer (pH 7.0; 50 mM, 20˚C) and saturated with 100 mmol PQQ. Subsequently, excess potassium ferricyanide and 0.1 mmol R,S-glycidol were added. When about 55% of the substrate had been oxidized the incubation reaction was stopped. The exact conversion was 56% and the enantiomeric excess of R-glycidol was 97%.

**Claims**

1. Enzymatic, enantioselective preparation of R-glycidol, characterized in that a mixture of R- and S-glycidol is treated with a suitable enantioselective oxidizing enzyme.

2. Process according to claim 1, characterized in that R-glycidol is prepared with an e.e. higher than 95%.

3. Process according to claim 1 or 2, characterized in that an alcohol dehydrogenase is used as enzyme.

4. Process according to claim 3, characterized in that a PQQ dependent alcohol dehydrogenase is used.

5. Process according to claim 4, characterized in that an alcohol dehydrogenase from the group of acetic acid bacteria is used.

6. Process according to claim 5, characterized in that the enzyme used is a PQQ dependent alcohol dehydrogenase originating from or obtained from the genus Acetobacter or the genus Gluconobacter.

7. Process according to claim 6, characterized in that the enzyme used is a PQQ dependent alcohol dehydrogenase originating from or obtained from Acetobacter pasteurianus, Acetobacter aceti or Gluconobacter oxydans.

8. Process according to claim 7, characterized in that the enzyme used is a PQQ dependent alcohol dehydrogenase originating from or obtained from Acetobacter pasteurianus ATCC 12874, Acetobacter aceti ATCC 15973 or Gluconobacter oxydans ATCC 621.

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US-A-4 764 628 (W.P.SHUM)<br>* claims *<br>--- | 1 | C12P41/00<br>C12P17/02 |
| A | EP-A-333 142 (LONZA)<br>* claims *<br>--- | 1 | |
| A | BIOCHEMICAL AND BIOPHYSICAL RESEARCH COMMUNICATIONS.<br>vol. 72, no. 3, 1976, DULUTH, MINNESOTA US<br>pages 1028 - 1034;<br>S.MCCAUL ET AL.: 'The reaction of epoxides with yeast glyceraldehyde-3-phosphate dehydrogenase '<br>* the whole document *<br>----- | 1 | |
| | | | **TECHNICAL FIELDS SEARCHED (Int. Cl.5)**<br><br>C12P |

The present search report has been drawn up for all claims

| Place of search | Date of completion of the search | Examiner |
|---|---|---|
| THE HAGUE | 08 OCTOBER 1991 | DELANGHE L.L.M. |